# EUROPEAN PATENT SPECIFICATION

(11) **EP 4 465 958 B1**
(45) Date of publication and mention of the grant of the patent: **29.10.2025**
(21) Application number: 23701454.3
(22) Date of filing: 20.01.2023
(51) Int. Cl.: A61K 9/00, A61K 47/32, A61K 31/565, A61K 31/57, A61K 45/06, A61P 15/18

(54) **A DRUG DELIVERY SYSTEM**
WIRKSTOFFFREISETZUNGSSYSTEM
SYSTÈME D'ADMINISTRATION DE MÉDICAMENTS

(30) Priority: 21.01.2022 EP 22152789
(43) Date of publication of application: 27.11.2024
(73) Proprietor: Sever Pharma Solutions, 212 15 Malmö (SE)
(72) Inventor: DE GRAAFF, Wouter, 212 15 Malmö (SE); VAN LAARHOVEN, Hans, 212 15 Malmö (SE)
(74) Representative: Holme Patent A/S
(86) International application number: PCT/EP2023/051383
(87) International publication number: WO 2023/139220

(56) References cited:
- WO-A1-2009/036999
- WO-A1-2015/086489

## Description

The present invention relates to a drug delivery system, a method of manufacturing said system and a dual drug delivery device comprising said system.

Various types of delivery systems have been developed for the controlled and sustained release of active ingredients preferably by diffusion through the surface of the device.

One such device is the intrauterine device (IUD); Mirena^{®} which is considered one of the safest and most efficient contraception used worldwide. In addition to preventing undesired pregnancies, the device provides several advantages: its use is controlled by the female; it allows for a better regulated dose of drug without attention by the user; and it avoids the destruction (by the intestine and by first pass through the liver) of an appreciable portion of the daily dosage of the drugs compared to their orally delivered counterparts.

Other devices commercially available today are the intra vaginal rings (IVRs), e.g. the Estring^{®}, Femring^{®}, and Nuvaring^{®}, or subdermal contraceptive implants, e.g. the Implanon^{®}, all of which provide controlled and sustained release of steroid molecules over a prolonged period, e.g. several weeks/months.

These known vaginal rings have been found particularly useful for the release of steroids, whose relatively small molecular size and substantially water-insoluble nature permit effective permeation through the hydrophobic polymer, such that therapeutic concentrations may be readily achieved in the body.

However, diffusion in polymers is complex and is known to depend on a number of different factors, e.g. temperature, the manufacturing process, the solubility and diffusivity of the drug in the polymer, the surface area of the drug reservoir, the distance the drug must diffuse through the device to reach its surface and the molecular weight of the drug.

Consequently, it remains a challenge to understand, predict and control the diffusion of small and large molecules in polymer systems. In this respect, the use of drug delivery device to deliver drugs requires a design that regulates the release rate so as to reliably provide the user with the appropriate daily dose throughout the lifetime of the device.

WO2009/036999 discloses drug delivery systems comprising (i) a drug-loaded thermoplastic polymer core layer, (ii) a drug-loaded thermoplastic polymer intermediate layer and (iii) a non-medicated thermoplastic polymer skin covering the intermediate layer, wherein said core layer is loaded with crystals of a first compound and the intermediate layer is loaded with crystals of a second compound.

In reservoir systems, i.e. a drug loaded core surrounded by a non-medicated membrane/sheath, the drug first partitions into the sheath from the reservoir and then diffuses to the other side of the sheath, where it is taken up by the receiving medium. While the reservoir is saturated, a constant concentration gradient of drug is maintained in the membrane, the rate of drug flux is constant, and zero order release is achieved. However, when drug concentration in the reservoir falls, the gradient across the membrane and the release rate of the drug also decreases.

Furthermore, reservoir systems can be difficult to fabricate reliably, and pinhole defects and cracks in the membrane surrounding the reservoir, can lead to dose dumping, **i.e.** unintended, rapid drug release over a short period of time.

These problems are avoided in monolithic systems, in which drug is loaded directly into a polymer, which now acts as both a storage medium and a mediator of diffusion. Drug is typically loaded uniformly into monolithic devices, and the release is controlled by diffusion through the monolithic matrix material or through aqueous pores. However, with passing time, release rate decreases, as drug that is deeper inside the monolith device must diffuse to the surface, since it has farther to travel, and the quadratic relation between distance and time becomes important. Since the geometric factor is essential in this respect, the effects can be minimized by using other geometric shapes or hemisphere monoliths to provide near-zero-order release, but such devices are neither easy nor inexpensive to fabricate.

Simultaneous drug delivery/release finds application in a number of different areas. However, the placement of a blend of the drugs in a single delivery device in a proportion equal to the desired delivery rate ratio will almost never achieve the desired result. In many cases, the drugs will not diffuse together through the surface or membrane at the same ratio, as they exist in the blend. The ratio would instead be dependent on the inherent ratio of the normalized permeation rates for the drugs through e.g. a rate-controlling membrane. Flexibility would therefore be limited to the selection of suitable polymer candidates for the sheath. Accordingly, the range of, and degree of control over, the delivery rate ratio, is extremely limited.

Of course the need for maintaining a specified delivery rate ratio can be met by using a separate delivery device for each drug. However, this is clearly undesirable, since the presence of two or more delivery devices will compound the disruption which even a single delivery device might create in the normal physiological activity of an animal or human. In addition, if one delivery device malfunctions, the desired delivery ratio will be lost. Further, complete therapy in a single implantable or insertable delivery device is more acceptable to patients and more efficient to insert and remove. Adjustment of a specified delivery rate can also be met by drug delivery systems comprised of several elements that each releases a drug at a specified rate. Examples are two or multi-compartments intravaginal rings, and ring bodies containing drug release capsules. The industrial scale manufacturing of such rings are however complex and expensive. A further problem with the known devices arranged for releasing more than one drug is that such devices usually show sub-optimum release patterns for the different drugs, whereas it is generally preferred that all drugs are released in a controlled rate during a specified duration of time. Furthermore, since each drug and delivery device combination behaves uniquely, the drug combination could have a significant impact on the drug(s) release characteristics. Thus, there is a demand for a novel drug delivery system arranged for releasing two active ingredients/drugs in a controlled manner and in the correct ratio, and a method for manufacturing the system that is simple and inexpensive.

Thus, it is a first aspect of the present invention to provide a drug delivery system, which can be loaded with two active ingredients and where each active ingredient is released at a controlled rate independently of the other active ingredient.

In a second aspect is provided a delivery system that reduces the variability of the release rate of active ingredients over time.

In a third aspect is provided a delivery system in which the known problems relating to complicated and expensive manufacturing processes, dose dumping and initial drug burst are eliminated, and which at the same time provides a substantially zero-order release rate of the active ingredient in the core.

In a fourth aspect is provided a delivery system which is stable at room temperature.

In a fifth aspect is provided a delivery system arranged for implantation e.g. subcutaneous or for vaginal or uterine placement in an animal or human.

The novel and unique features whereby these and further aspects are achieved according to the present invention is by providing a drug delivery system comprising a core comprising a one first polymeric material and a first active ingredient, and a sheath comprising a second polymeric material and a second active ingredient dispersed and/or incorporated in the second polymeric material in a concentration of at least 10 wt% based on the weight of the sheath, wherein the first active ingredient and second active ingredient is a steroid, and wherein the steroid is a contraceptive agent, and wherein the sheath is the outer layer of the drug delivery system.

The drug delivery system according to the invention relates to a system that comprises a core surrounded, at least partly but preferably completely, by a sheath/membrane. However contrary to the conventional systems of this kind, the sheath of the present invention also comprises a high concentration of an active ingredient, whereby a simultaneous release of two active ingredients is provided.

Within the context of the present invention the term "active ingredient" means a substance intended to be released to a surrounding medium where it will have an effect in the diagnosis, cure, mitigation, treatment or prevention of disease, or an effect in restoring, correcting or modifying physiological functions in a subject, e.g. an animal or human.

Dual administration finds application in a number of different areas, e.g. in contraceptive rings and in dual delivery systems, e.g. implants and rings providing e.g. hormone replacement therapy or contraception and protection against infections, e.g. HIV-infection.

For such dual administration systems it is required to release the two active ingredients simultaneously and at the same time. It is therefore necessary to adjust the release rate of these drugs independently to the physiological optimal rate (mg/day). Using the drug delivery system according to the present invention the inventors have found that it is possible to attain independent and optimal release of two active ingredients without the need for complex assembly of parts and without the need to use sophisticated multi-layer extrusion technology.

Using a drug delivery system in which the concentration of the active ingredient in the sheath is at least 10wt% it is ensured that the desired near zero-order release behavior of the active ingredient in the core are observed for a longer period of time. In addition such high concentrations of active ingredient are associated with good physical stability of the relevant active ingredient.

By substantially zero order is meant that a substantially constant amount of the relevant active ingredient is released over a given period of time. In some embodiments, the system exhibit a substantially zero order release profile of the first active ingredient(s) over a treatment period of at least one month, preferably at least two month, and even more preferred more than three months, or even longer e.g. up to one year.

As discussed earlier it is a well known problem that the release rate of the active ingredient in the core decreases as active ingredient(s) that is deeper inside the core/reservoir must diffuse to the surface. However loading the sheath with a high concentration of active ingredient the inventors of the present invention have found that the release rate of the first active ingredient in the core slightly increases over time, thereby compensating for the further distance said active ingredient must travel to the surface of the delivery system for being released to the surroundings, thereby providing a substantially zero-order relate rate of the first active ingredient during the desired treatment period.

It is important that the second active ingredient is dispersed and/or incorporated in the second polymeric material to an extent sufficient to control the diffusion rate of the first active ingredient through the sheath, while the reduced diffusion rate is still sufficient to maintain an effective level of active ingredient on the surface of the drug delivery system according to the invention.

Without being bound by theory, it is believed that the second active ingredient in the sheath will function as a filler and control the release rate of the first active ingredient. When the second active ingredient present in the sheath is released to the surroundings, the concentration of said second active ingredient is reduced and it is believed that that diffusion of water into the sheath may be facilitated leaving behind an empty porous matrix and/or empty pockets/holes and/or the sheath may collapse thereby ensuring the desired zero-order release profile of the first active ingredient. Thus, it is believed that the space initially occupied by the second active ingredient leaves behind an empty porous matrix which may become water filled due to ingress of water and/or may leave behind empty pockets/holes. This is contrary to the conventional findings in which the release rate of an active ingredient in a core surrounded by a non-medicated sheath slightly decreases over time, i.e. the desired zero order release rate cannot be maintained over a desired treatment period for such conventional systems.

In some embodiments it is preferred that at least 15 wt% of the second active ingredient is dispersed in the second polymeric material, even more preferred at least 20 wt%, and even more preferred at least 25 wt% or even higher. The exact amount of the second active ingredient will depend on the used polymer(s) and active ingredient(s).

The presence of the second active ingredient in the relatively high concentrations will not only lead to an increase in the mean path length the molecules of the first active ingredient have to travel between two points in the sheath, but will also reduce the amount of the first active ingredient which can be dissolved in the polymeric material of the sheath, accordingly decreasing the release rate of the first active ingredient though said sheath. This will provide a reliable release rate and a lower initial burst of the first active ingredient. Accordingly, the sheath can be made smaller, providing a smaller product with a significant lower burst of the first active ingredient.

It is in this respect preferred that the second active ingredient is incorporated and/or dispersed in the second polymeric material in the form of particles, preferably crystals. Such particles/crystals will form a repository of solid, undissolved crystals which will act as seed crystals i.e. as a slow release depot. Over time, when the second active ingredient is delivered to the surroundings, some of the crystals will be unlocked in the second polymeric material, thereby providing a prolonged release of the second active ingredient. Furthermore, the stability of the second active ingredient in the drug delivery system is improved when the active ingredient is incorporated into the sheath as undissolved particles/crystals.

In order to obtain the desired zero order release profile of the first active ingredient during the treatment period, it is preferred that the first active ingredient is dispersed and/or incorporated in the first polymeric material of the core in a concentration of at least 5 wt% of the total weight of the core, preferably at least 10 wt%, preferably at least 15 wt% and even more preferred at least 20 wt%. However, the concentration of the first active ingredient may also be higher such as at least 30 wt% of the of the total weight of the core.

It is preferred that the first active ingredient also is present in the core in the form of particles, preferably crystals, for the same reasons as disclosed for the second active ingredient. In this way the stability of both the first and second active ingredient, and accordingly the drug delivery system according to the intention, is improved.

Alternatively, the first active ingredient can be dissolved in the first polymeric material and in these embodiments the first active ingredient is preferably present in the first polymeric material at a concentration below the saturation concentration of said first active ingredient at a temperature of 25°C.

Since it is expected that some of the first active ingredient will/may redistribute throughout the intravaginal ring upon storage, i.e. the concentration of first active ingredient in the core will drop as a part of the first active ingredient will diffuse into the sheath, the term "below the saturation concentration" refers to the concentration of the first active ingredient in the core measured in an equilibrated drug delivery system, i.e. when an equilibrium of the first active ingredient has been reached in the system.

Without being bound by theory it is believed that maintaining a concentration of the second active ingredient at high concentrations, a porous network path is created by crystals and wherein a number of sites/openings/pores in the matrix of the polymeric material remains empty, ensuring that the active ingredient only can be released through a tortuous path in the sheath, thereby the diffusion length increases and the release rate is controlled.

However, in order to obtain a desired release rate of the first active ingredient it is preferred that the concentration of the second active ingredient in the second polymeric materials does not exceed the percolation threshold of said second active ingredient in the sheath.

Percolation theory can be applied to inert matrix systems in which the sites/openings/pores of the material of the matrix are occupied at random by particles of a specific component. When the particles occupy the neighboring sites in the matrix a cluster is formed, and when this cluster percolates the whole matrix, it is considered to be a sample-spanning cluster i.e. an infinite or percolating cluster. The concentration of component at which there is a maximum probability of appearance of a sample-spanning cluster of this component is named percolation threshold, see Millán M, Caraballo I, Rabasco A.: The role of the drug/excipient particle size ratio in the percolation model for tablets. Pharm Res. 1998;15(2):220-224. Thus, simply speaking, the percolation threshold corresponds to the minimum concentration of the respective active ingredient at which an infinite cluster spans the matrix.

Applied to the present invention, the matrix is formed by the second polymeric material, and when a cluster formed by particles of the second active ingredient provides a continuous phase through the matrix of the sheath, the percolation threshold is reached. It is believed, without being bound by theory, that at or above the percolation threshold the effective diffusion length would decrease, since the cluster of active ingredient will be connected with the surface, and the release rate of the active ingredient would increase.

The percolation threshold depends among others of the particular active ingredient, the polymeric material used, and the size of the core and/or sheath of the drug delivery system according to the invention. However, those skilled in the arts will be able to determine the percolation threshold for any given active ingredient(s) in a drug delivery system according to the present invention in accordance with standard procedures, e.g. the procedures disclosed in e.g. Pharmaceutical Research, Vol. 23, No. 10, October 2006; AAPS PharmSciTech, Vol. II, No.2, June 2010; and/or Pharmaceutica Acta Helvetiae, Vol. 68, issue 1, July 1993, pages 25-33.

In an alternative embodiment the concentration of the concentration of the second active ingredient in the second polymeric materials is 40 wt% or below based on the weight of the sheath, and preferably 35 wt% or below.

In a similar manner the concentration of the first active ingredient in the first polymeric material should be below the percolation threshold of said first active ingredient in the core, or alternatively the concentration of the first active ingredient in the first polymeric materials should be 40 wt% or below based on the weight of the core, e.g. 30 wt% or below.

It must be noted that even though it is known to have small concentrations of active ingredient in a sheath surrounding a drug loaded core, see e.g. WO2013/120888, it is not known to have an active ingredient in the sheath in the high concentrations claimed in the present invention. Small concentrations of active ingredient i.e. concentrations well below 10 wt%, will have no or only a very limited effect on the release rate of the active ingredient in the core, and are accordingly not relevant for the present invention.

A person skilled in the art will based on the context of the present invention understand that it will be possible to control and/or adjust the diffusion rate through the sheath by varying the amount/concentration of the second active ingredient in said sheath, by using different polymeric materials, and/or by using different particle sizes of the second active ingredient or blends of different particles sizes.

It is however preferred that the particles/crystals of the second and optionally the first active ingredient have a mean particle size of between 3 µm and 40 µm, preferably between 8 µm and 24 µm, and even more preferred between 10 and 24 µm as it has been proven that such particles sizes provide the desired near zero order relate rate for a prolongs period of time, i.e. for at desired treatment period of at least one month.

As used herein, the term "crystals" refers to particles of the active ingredient arranged in an ordered microscopic structure, forming a crystal lattice. The term "crystal size" or "particle size" refers to a crystal's or particle's mean particle diameter. Particle size, crystal size and/or particle size distribution is preferably be measured using laser diffraction, e.g. using a Malvern laser scattering particle size analyzer. However any other particle size measurement apparatus or technique known to person's skill in the art can also be used, e.g. dynamic light scattering, or a sieve analysis. As used herein, the term "crystal size" or "particle size" relates to the particle distribution diameter of the particle/crystal. As an example can be mentioned that D90 means that 90% of the particles have a diameter below the given value when measured using e.g. laser diffraction, dynamic light scattering, or a sieve analysis.

The polymeric materials used in the drug delivery system of the present invention are preferably suitable for subcutaneous insertion/implantation, or for placement in the uterus or vaginal tract, i.e. the materials are non-toxic and non-absorbable in a patient or animal. In this respect a variety of inert thermoset or thermoplastic elastomer, as well as combinations of polymeric materials are contemplated within the scope of the present invention.

In one embodiment the first and/or second polymeric material is a silicone polymer (thermosetting type). Silicone elastomers, such as poly (dimethylsiloxane) are already used conventionally for IVRs and similar silicones are also contemplated within the scope of the present invention.

It is however preferred that the first and second polymeric material is a thermoplastic polymer, which in principle can be any extrudable thermoplastic polymer material suitable for pharmaceutical use, such as ethylene-vinyl acetate (EVA) copolymers, low-density polyethylene, polyurethanes, and styrene-butadiene copolymers.

In one embodiment, ethylene-vinyl acetate (EVA) copolymer is used as both the first and second polymeric material due to its excellent mechanical and physical properties.

The vinyl acetate concentration of the EVA-polymer determines the rate of diffusion of the active ingredient through the system and generally, the lower the vinyl acetate concentration, the slower the active ingredient will be release from the polymer or migrate through it.

It is in this respect preferred that the second polymeric material of the sheath is a ethylene-vinyl acetate copolymer with a vinyl acetate content from 12 to 28 wt%, preferably a vinyl acetate content between 14 and 24 wt%, such as around 20 wt%, as these materials will provide the desired release profile through the second polymeric material, i.e. through the sheath.

In order to provide the desired zero order release profile of the first active ingredient it is preferred to have a higher release rate through the core, such that the sheath becomes the rate limiting factor. It is accordingly preferred that the first polymeric material of the core is an ethylene-vinyl acetate copolymer with a vinyl acetate content from 26 to 40 wt%, preferably 28 wt%, 33 wt% or 40 wt%.

When a specific vinyl acetate content e.g. 20 wt% is mentioned it refers to the weight% content provided by the manufacture. However, manufactures may use different internal analytical methods for determining vinyl acetate content, and there may therefore be variations in the range of 1 - 2 % in the actual vinyl acetate content depending on the manufacture. Thus, in the present invention the vinyl acetate content refers of the vinyl acetate content in the ethylene-vinyl acetate copolymer determined by high resolution NMR according to standard methods. The wt% of the vinyl acetate content in the ethylene-vinyl acetate copolymer is the wt% content based on the weight of the ethylene-vinyl acetate copolymer.

The drug delivery system according to the invention is a dual-drug delivery system i.e. it comprises two active ingredients, wherein the first active ingredient and second active ingredient is a steroid, and wherein the steroid is a contraceptive agent, which can be administrated subcutaneously, subdermal, vaginally or to the uterus.

In a preferred embodiment the first and second active ingredient are different steroids, e.g. different contraceptive agents such as an estrogenic steroid, and/or a progestational steroid. In a preferred embodiment the first active ingredient is estradiol and the second active ingredient is a progestogen selected from the group consisting of levonorgestrel, etonogestrel, d-1-norestrel and norethindrone, preferably levonorgestrel. However the steroids can also be selected in order to treat other conditions, e.g. vaginal atrophy and symptoms associated with menopause, e.g. hot flashes.

Irrespectively of the first and second active ingredient or the intended use of the device, the drug delivery system according to the invention is adapted to deliver pharmaceutically effective amounts of active ingredient(s). By "pharmaceutically effective," it is meant an amount, which is sufficient to affect the desired physiological or pharmacological change in the subject. This amount will vary depending upon such factors as the potency of the particular ingredient, the desired physiological or pharmacological effect, and the time span of the intended treatment. Those skilled in the arts will be able to determine the pharmaceutically effective amount for any given active ingredient(s) in accordance with standard procedures.

The thickness of the sheath, which is the outer layer of the drug delivery system according to the present invention, can be varied to further control the release rate of the active ingredients. Thus, the drug delivery system according to the invention does not contain/comprise any sheath/membrane (e.g. a rate-controlling sheath) without an active ingredients.

In one embodiment the thickness of the sheath is between 0.05 mm and 3 mm. Said thickness is preferably between 0.05 mm and 2 mm, more preferably between 0.1 mm and 2 mm and even more preferably between 0.2 mm and 0.6 mm, depending on the active ingredient(s) and the polymeric material. In certain embodiments the thickness of the sheath is 120 µm, 240 µm or 320 µm.

A person skilled in the art will in view of the present invention understand that a thin sheath can contain less active ingredient than a thicker sheath; and that the concentration of the second active ingredient in the sheath should be enough to sustain release at the desired rate of the relevant active ingredients over the desired treatment period. By using an ethylene-vinyl acetate copolymer grade with higher or lower vinyl acetate-content the sheath's thickness can be altered while maintaining essentially the same average release rate of the first active ingredient. For instance, if a thicker skin is desired because more of the second active ingredient has to be accommodated in the sheath, an ethylene-vinyl acetate copolymer grade with higher vinyl acetate content can be chosen.

In a similar manner the core preferably has a round cross-section with a cross-sectional diameter of between 2 and 8 mm, more preferably between 3 mm and 6 mm and even more preferably around 4 mm.

The drug delivery system according to the invention is preferably in the form of, or formed into, or part of an implant, an intrauterine device or vaginal ring.

In a preferred embodiment, the system of the present technology is an intravaginal ring (IVR). The dimensions of the IVR may vary depending upon the anatomy of the subject, the amount of drug to be delivered to the patient, the time over which the drug is to be delivered, the diffusion characteristics of the drug and other manufacturing considerations. The only requirement being that the IVR should be flexible enough to enable bending and insertion inside the vaginal cavity and rigid enough to withstand the expulsive forces of the vaginal musculature without causing abrasion to the vaginal epithelium. The outer diameter of such IVRs may range, e.g., from about 45 mm to about 65 mm, and/or the length of the fibers forming the IVR may have a length from 150 to 170 mm, preferably from 154 to 160 mm, such as about 157 mm.

In the context of the present invention the term intravaginal ring, also contemplates ring designs or structures, which have other shapes, e.g. polygonal shapes and/or wavy shapes, or where the structure is not a complete and/or closed circle/shape.

In a preferred embodiment according to the present invention the drug delivery system does not comprise more than the two active ingredients, i.e. the first and second active ingredients, and/or does not comprise further cores and/or layers such as sheaths and membranes. Thus in a preferred embodiment the drug delivery system according to the invention consists of a single core and a single sheath completely surrounding said core, and wherein the first active ingredient is part of the core and the second active ingredient is part of the sheath.

The present invention also relates to a method of manufacturing the drug delivery system according to the present invention.

Said method comprises
a. providing a core comprising a first polymeric material and a first active ingredient, and
b. providing a sheath comprising a second polymeric material and a second active ingredient dispersed and/or incorporated in the second polymeric material in a concentration of above 10wt% based on the weight of the sheath, and
c. co-extruding the core and sheath into a fiber.

In order to form the drug delivery device, the fibers obtained in step c. may e.g. be cut into appropriate length, and formed into an implant, an IVR using conventional techniques, or by combining them with other elements for forming an IUD.

As the core and sheath are co-extruded a very simple and inexpensive embodiment according to the invention is provided, however the cores and sheath can be formed in separate injection molding or extrusion steps if preferred. Injection molding and extrusion are well known in the art and will not be discussed further in this application.

It is preferred that the method further comprises a cooling step in which the provided drug delivery system is cooled to a temperature of 20°C or below for providing the crystals in the sheath and optionally the core. This may e.g. be obtained by placing the fiber into a cooling water bath.

Without being bound by theory, the inventors believe that the crystals formed in the sheath and optionally the core is caused by the kinetics of re-crystallization. The relatively high concentrations of active ingredients in both the core and sheath load will result in a higher concentration of "seed" crystals, upon which recrystallization can occur when the fiber is cooled after co-extrusion.

It is preferred that said cooling step is performed immediately after step c., i.e. as fast as is practically possible from a production point of view, i.e. preferably within less than 30 minutes from completion of the fiber in step c.

The invention will be explained in greater detail below, describing an exemplary embodiment of the drug delivery system according to the invention
Fig. 1 shows a perspective view of a preferred embodiment of a vaginal ring according to the invention,
Fig. 2 shows photos of a cross-section of the fibers with a 0.39 wt% estradiol loaded core and 5 wt% progesterone loaded sheath (not according to the claims) with a sheath thickness of (a) 200 µm; (b) 300 µm, and (c) 400 µm,
Fig. 3 shows photos of a cross-section of the fibers with a 0.39 wt% estradiol loaded core and 33.9 wt% progesterone loaded sheath with a sheath thickness of (a) 200 µm; (b) 300 µm and (c) 400 µm,
Fig. 4 shows photos a cross-section of the fibers with a 10 wt% estradiol loaded core and 33.9 wt% progesterone loaded sheath with a sheath thickness of (a) 200 µm; (b) 300 µm and (c) 400 µm,
Fig. 5A and B shows progesterone and estradiol release of IVRs with 0.39 w/w % estradiol in the core and 5 w/w% progesterone in the sheath (not according to the claims),
Fig. 6A, B and C shows progesterone release of different IVRs,
Fig. 7 shows progesterone release of different IVRs with 10 w/w % estradiol in the core and 33.9 w/w% progesterone in the sheath,
Fig. 8 shows estradiol release of different IVRs with different estradiol and progesterone concentrations in core and sheath, and
Fig. 9 shows estradiol release of different IVRs.

The invention is described with the assumption that the drug delivery system is a vaginal ring. However, this assumption is not to be construed as limiting, and the system could just as easily have a different structure/design, e.g. be an IUD, such as a hormone spiral, or an implant.

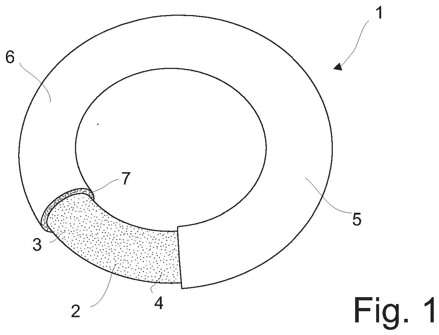

Fig. 1 shows a preferred embodiment of an intravaginal ring (IVR) 1 according to the invention. In said embodiment the IVR has a reservoir design i.e. it comprises a core (2) made of a first polymeric material (3) and comprising a first active ingredient (4), and a sheath (5) made of a second polymeric material (6) and comprising a second active ingredient (7) dispersed and/or incorporated in the second polymeric material in a concentration of above 10 wt% based on the weight of the sheath.

### Example 1

In order to evaluate the effect of estradiol (E2) concentration in the core; progesterone (P4) concentration in the sheath, and the effect of the sheath thickness on the release rate of estradiol and progesterone respectively, a series of intra-vaginal rings (IVRs) was prepared as follows.

### Raw materials used in the production

| **Material** | **Supplier Batch number** | **Supplier** | **Comments** |
|---|---|---|---|
| Micronized Progesterone | J44723 | Pfizer | Particle size average EP: 5 mm |
| | | | Particle size count LT: 5 mm: 85% |
| | | | Particle size count LT: 10 mm: 100 % |
| Micronized Estradiol | - | Aspen Oss | Particle size: min 90% ≤5 mm and min99 % ≤15 mm. |
| Milled EVA 28 | Ateva (R) 2820A EVA copolymer | Celanese | Melt flow index: 22.10 g/10 min |
| | | | Vinyl acetate content: 28.5 % |
| Magnesium stearate (Mgst) | - | Peter Greven, Venlo | - |

### Process schema and production

The core/sheath fibers containing the API's estradiol and progesterone were prepared as shown in the following process flow for fiber production.

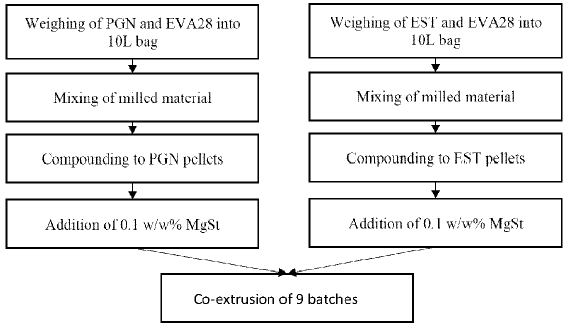

The milled EVA and APIs were weighed according to the proportion detailed in table 3; all four batches are mixed using the same mixing protocol. The compounding is conducted using an 11 mm closely intermeshing twin screw extruder (Pharma 11 twin screw extruder from Thermo scientific). In summary, four batches were compounded according to table 3 to produce active granulate/pellets. The compounding set temperature of all batches is 90 °C.

**Table 1: Summary of batches of active materials.**

| **Batch number** | **EST % (w/w)** | **PGN % (w/w)** | **EVA 28 %(w/w)** |
|---|---|---|---|
| **Purpose** | For fiber's core | For the fiber's sheath | For the sheath and the core |
| **A** | 0.39 | - | 99.61 |
| **B** | - | 5 | 95 |
| **C** | - | 33.9 | 66.1 |
| **D** | 10 | - | 90 |

To enhance the processing properties of the pellets in the co-extrusion process, magnesium stearate (Mgst) was added to all batches. First, the 0.1 wt% of Mgst was added to the mixing bags with active pellets and then manually mixed for around 3 minutes.

A co-extruder line with a 5 mm die was used to produce the 5 mm sheath/core fibers. The co-extruder line comprises a single screw extruder 16/25 (D/L) i.e. 16 is the diameter and 25 is the length over diameter ratio (D/L)) for the sheath and a single screw 25/25 (D/L) for the core.

Both the core and the sheath of the produced fibers contain active ingredients; in addition, the sheath thickness is varied for each formulation to obtain 200 µm, 300 µm, and 400 µm sheath thickness in 5 mm fiber diameter, as shown in Table 5.

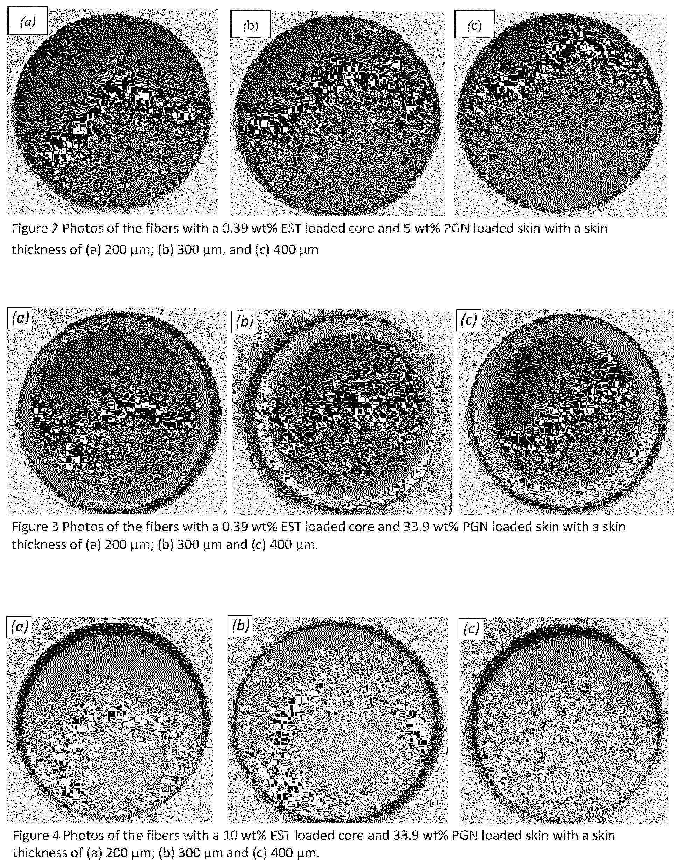

The fiber characteristics (diameter and sheath thickness were controlled by the melt pump speed for extruder 1 (sheath) and extruder 2 (core) to produce a fiber of 5 mm diameter with the required sheath thickness. All the fibers were extruded between 90 °C and 100 °C. The melt pump speed was calculated based on the capacity of the pumps, and the sheath thickness compared to the core can be seen in Figure 3 (AC-batch) and 4 (DC-batch). No boundary could be seen for the AB-batch (Figure 2). The melt pump speed 1 and 2 for each batch are given in Table 2.

**Table 2: Melt pump speed for each formulation Melt pump volume for the sheath is 0.6 cm³ and 2.4 cm³ for the core**

| | Sheath thickness (µm) | Melt pump sheath (rpm) | Melt pump core (rpm) | Designation |
|---|---|---|---|---|
| 0.39 % EST (core) - 5 % PGN (sheath) | 200 | 12,146 | 15,125 | AB200 |
| | 300 | 17,983 | 13,813 | AB300 |
| | 400 | 23,561 | 12,559 | AB300 |
| 0.39 % EST (core) - 33.9 % PGN (sheath) | 200 | 12,875 | 15,125 | AC200 |
| | 300 | 19,054 | 13,813 | AC300 |
| | 400 | 24,959 | 12,559 | AC400 |
| 10 % EST (core) - 33.9 % PGN (sheath) | 200 | 12,875 | 15,442 | DC200 |
| | 300 | 19,054 | 14,103 | DC300 |
| | 400 | 24,959 | 12,824 | DC400 |

After co-extrusion, the fibers are cut into a length of 157 mm and then welded to form the intra-vaginal rings (IVRs). The produced IVRs (batches) are given in Table 3.

For the AB batch the progesterone concentration loaded in the sheath is 5wt%, and during extrusion the progesterone is as anticipated completely dissolved. As a consequence of internal diffusion progesterone will redistribute throughout the ring and in case no premature crystallization will take place the progesterone concentration in the ring will be fully homogeneous once the ring is in equilibrium.

**Table 3: Produced hatches together with the API content**

| **Batch*** | **w/w% EST** | **mg EST / ring** | **w/w% PGN** | **mg PGN / ring** | **Fiber diameter** |
|---|---|---|---|---|---|
| AB200 | 0.39 | 9.7 | 5.0 | 22.6 | 5.00 |
| AB300 | 0.39 | 8.8 | 5.0 | 33.3 | 5.00 |
| AB400 | 0.39 | 8.0 | 5.0 | 43.4 | 5.00 |
| AC200 | 0.39 | 9.7 | 33.9 | 162.5 | 5.00 |
| AC300 | 0.39 | 8.8 | 33.9 | 238.6 | 5.00 |
| AC400 | 0.39 | 8.0 | 33.9 | 311.4 | 5.00 |
| DC400 | 10 | 210.4 | 33.9 | 311.4 | 5.00 |
| DC 300 | 10 | 231.0 | 33.9 | 238.6 | 5.00 |
| DC 200 | 10 | 252.4 | 33.9 | 162.5 | 5.00 |

| | | | | | |
|---|---|---|---|---|---|
| *The numbers 200,300, and 400 stand for 200 µm, 300 µm, and 400 µm sheath thickness, respectively. | | | | | |

Batches AB200, AB300 and AB400 are not according to the claims.

### Optical observation of fibers

For batches AB200, AB300, and AB400, i.e. 0.39 wt% estradiol and 5 wt% progesterone, no clear boundaries between the core and sheath could be observed as shown in Figure 2, contrary to the other batches, shown in Figure 3 and 4. This is because estradiol and progesterone were dissolved in EVA 28 in the sheath and core, respectively.

The contrast (i.e., boundary) between the core and sheath is maximal for dissolved estradiol (0.39 wt%) and crystal loaded progesterone (33.9 wt%) for batches AC200, AC300, and AC400, cf. Figure 3.

The contrast (i.e., boundary) is low when both APIs are present in crystal form, i.e. for the IVRs comprising 10 wt% estradiol and 33.9 wt% progesterone (in addition to a dissolved fraction of API), as seen in the case of DC200, DC300, and DC400, see Figure 4.

The observed sheath/core system generally showed a centered/concentred geometry, as shown in Figure 3 and 4.

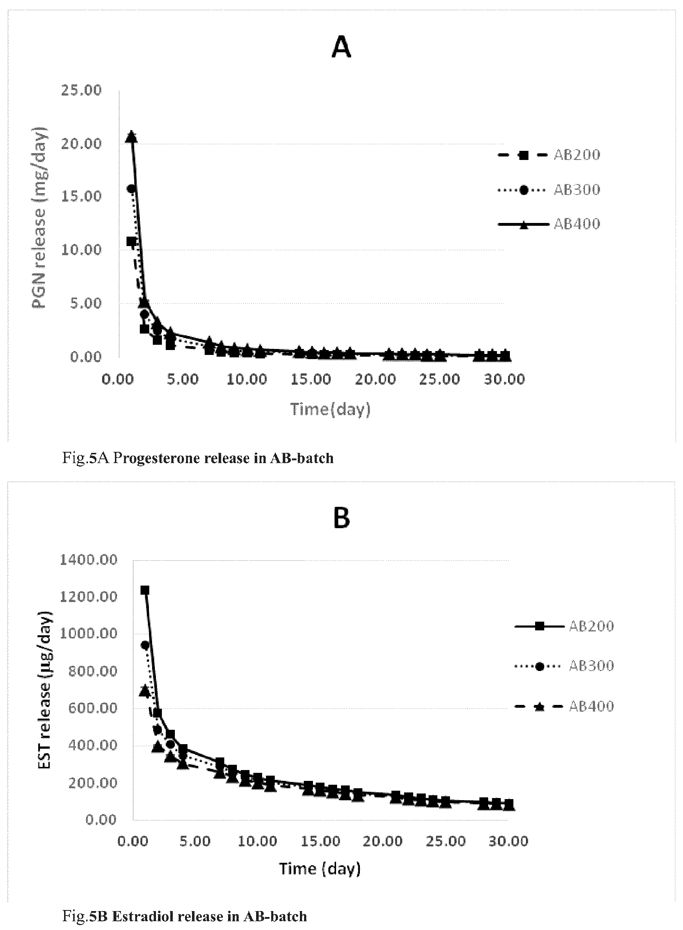

The release rate of estradiol and progesterone of the prepared IVRs are given in table 4, which shows the estradiol data, table 5, which shows the progesterone data, and the corresponding figures 5 - 9.

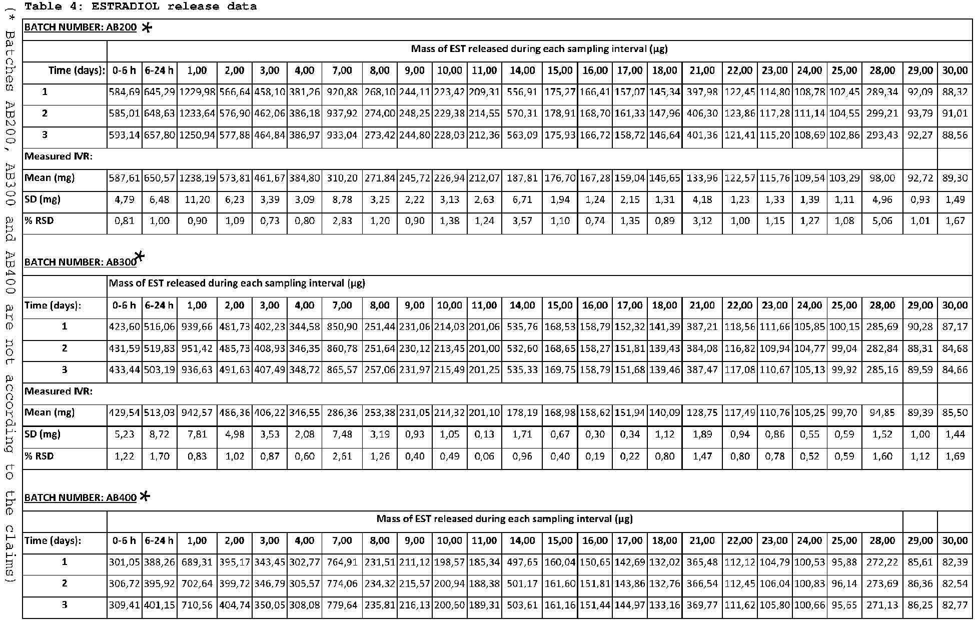

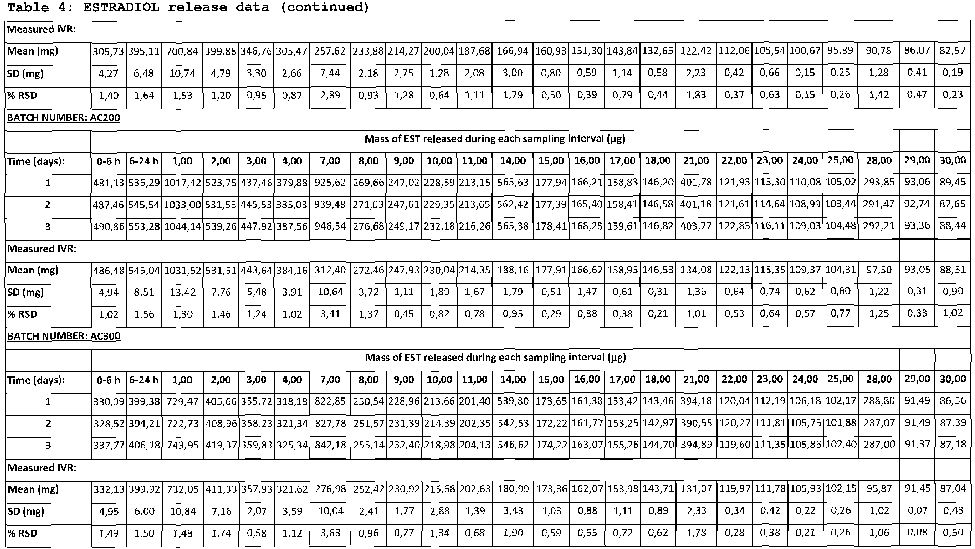

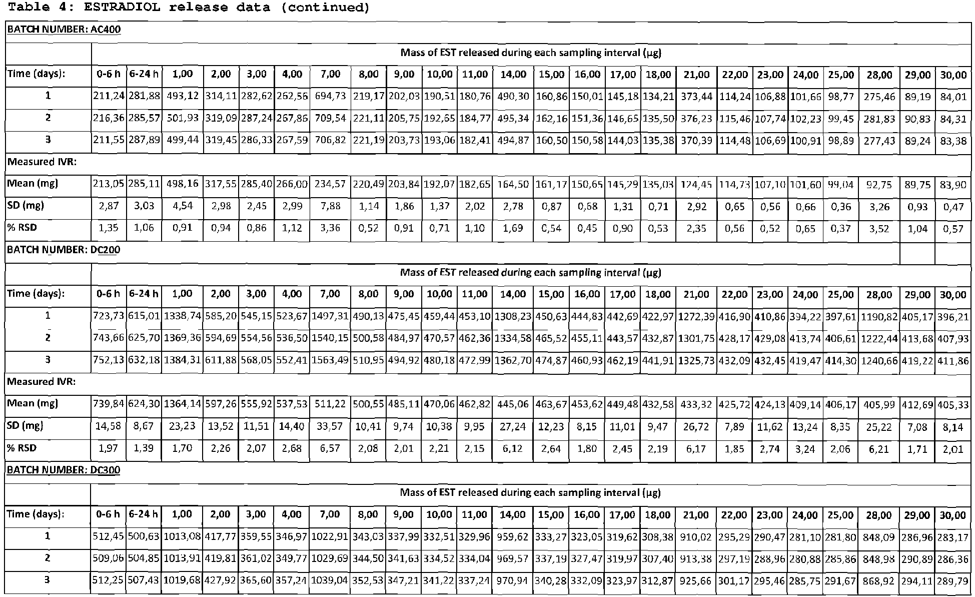

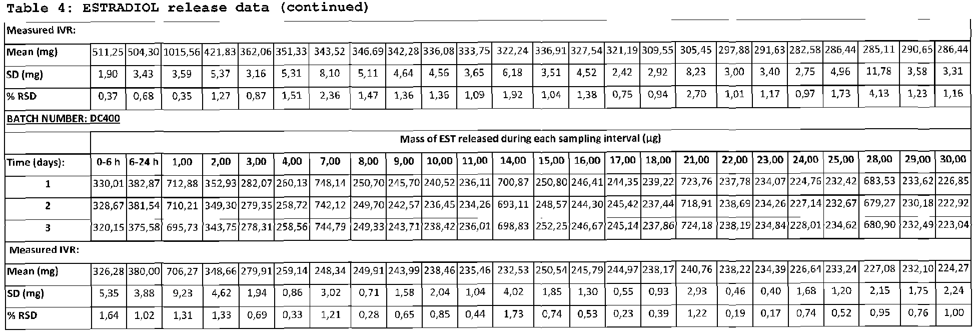

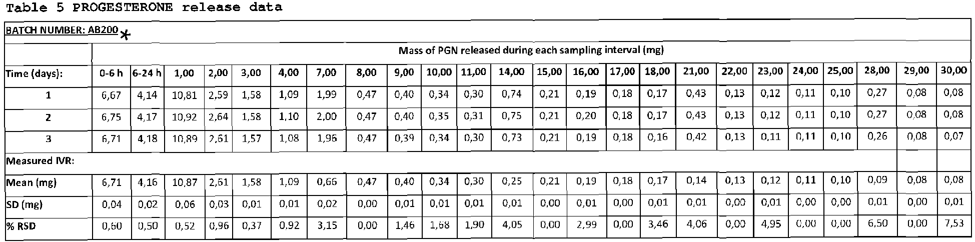

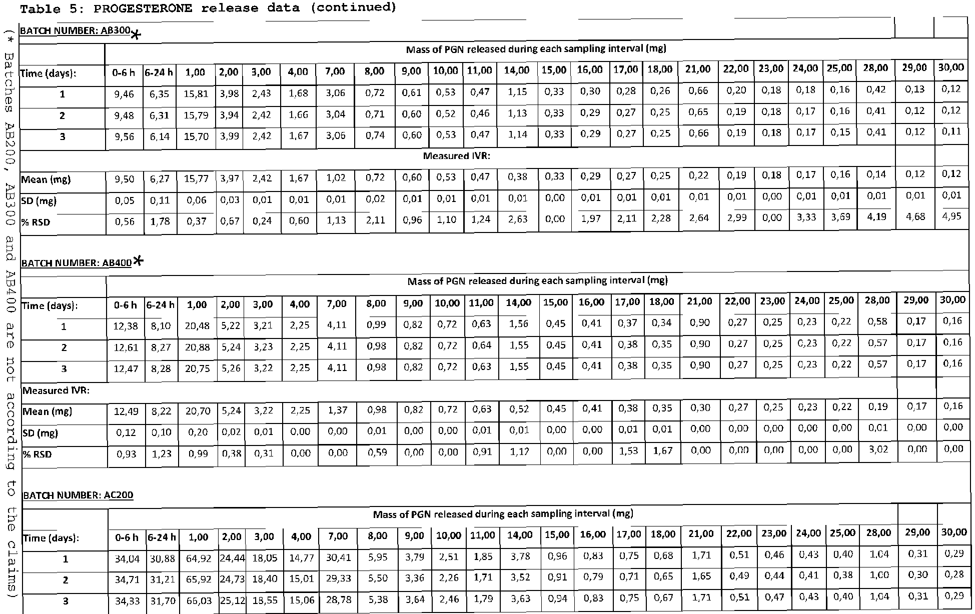

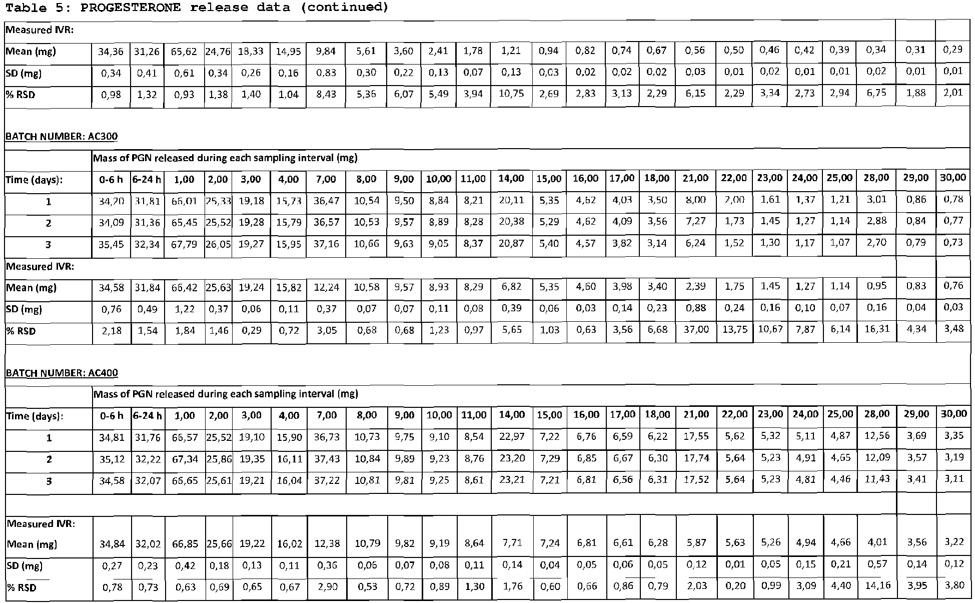

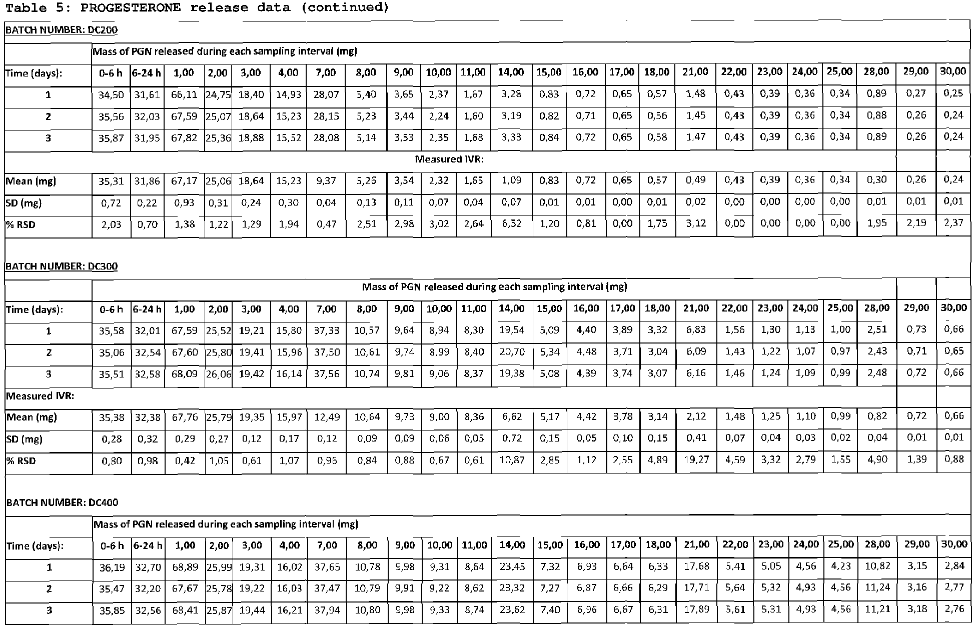

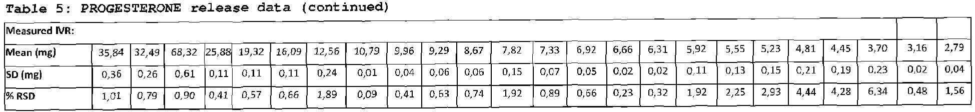

The results from table 4 and 5 will be discussed in further details below.

### Release of Estradiol and Progesterone in the provided IVRs

### IVRs comprising 0.39 wt% estradiol and 5 wt% progesterone with different sheath thickness (AB-batch, not according to the claims)

The release of estradiol and progesterone from IVRs obtained from batches containing 0.39 wt% estradiol and 5 wt% progesterone, i.e. the IVRs AB200, AB300 and AB400 are shown in Figure 5A and 5B.

As is evident from said figures the release of progesterone increases with increasing fiber sheath thickness; for all the cases, the daily release is less than 1 mg/day after day 10.

As estradiol is only present in the AB-batches in a low concentration (0.39 wt%) the estradiol is dissolved in the ring, and since the sheath and core are made from the same polymer (no partitioning) as for the AB200, AB300, and AB400 batch (see fig. 10), the estradiol concentration in equilibrium will be uniform and hence not affected by sheath thickness. However, a slight effect can be seen in fig. 5B and it is expected that this is attributed to the fact that small amounts of progesterone crystals are increasing the effective diffusion path, however only insignificantly.

Thus it is clear that small concentrations (5 wt%) of progesterone in the sheath have no or only a very limited effect on the release rate of the estradiol in the core.

### Release of progesterone in dependence of estradiol concentration in the core

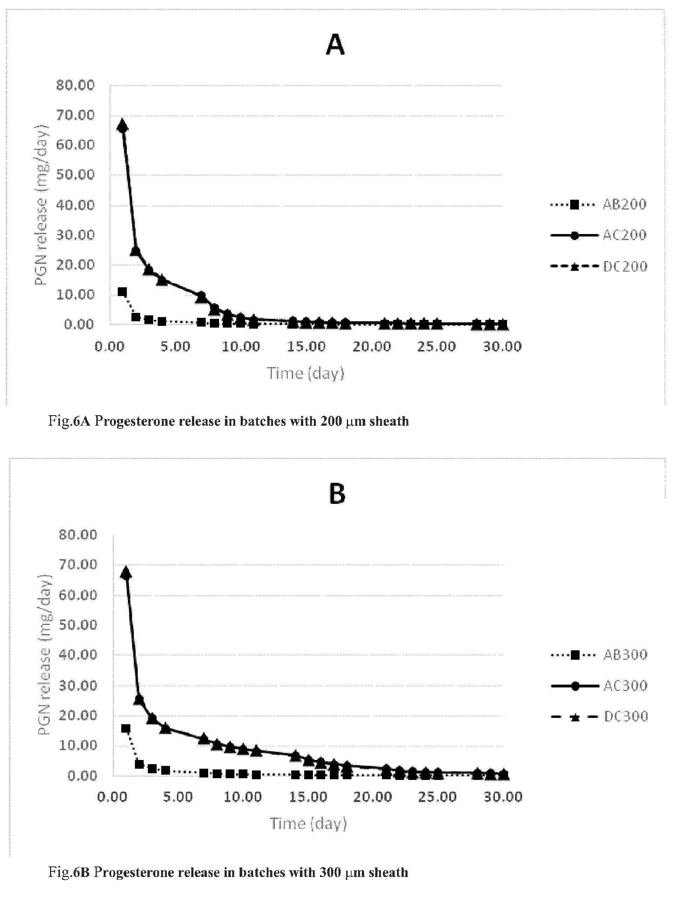

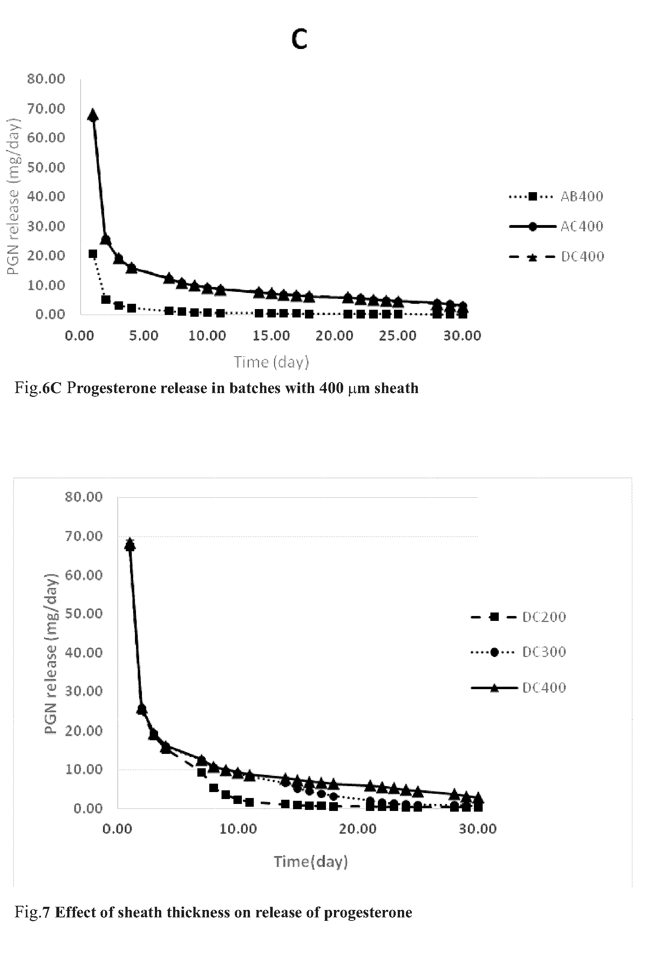

A comparison of the release rate of progesterone in the sheath in dependence of concentration of estradiol and sheath thickness is shown in Figure 6A, 6B and 6C.

As is evident from said figures, the release of progesterone is much higher in the IVRs having 33.9 wt% progesterone in the sheath compared to the IVRs having 5 wt% progesterone in the sheath.

However as can be seen in Figure 6A, B and C data it can also be concluded that the presence of estradiol in either dissolved, i.e. in a relatively low estradiol concentration (0.39 wt%), and/or in a crystal state i.e. a higher estradiol concentration (10 wt%), does not affect the release of progesterone. Note that as ring containing crystalline estradiol will also contain estradiol in a dissolved state and likely equal or close to the saturation concentration.

For instance in Figure 6A the IVRs AC200 (0.39 wt% estradiol) and DC200 (10 wt% estradiol) have substantially identical release profiles of progesterone, and the same can be seen for AC300 (0.39 wt% estradiol) and DC300 (10 wt% estradiol) in Figure 6B and AC400 (0.39 wt% estradiol)and DC400 (10 wt% estradiol) in Figure 6C.

### Effects of sheath thickness on the release of progesterone

As is shown in Figure 7 the increase in the sheath thickness allows sustained delivery of progesterone.

The daily release of progesterone from both AC and DC batches (for the same sheath thickness) is the same until the phenomenon of depletion is significant for each sheath thickness. Early on day 7, the impact of depletion on IVR is visible on DC200 (AC200), whereas DC300 (AC300) occurs on days 12-13. Even though only the data for the DC200 batches are shown in Figure 7, the data are identical for the AC batches.

### Effects of sheath thickness on the release of estradiol

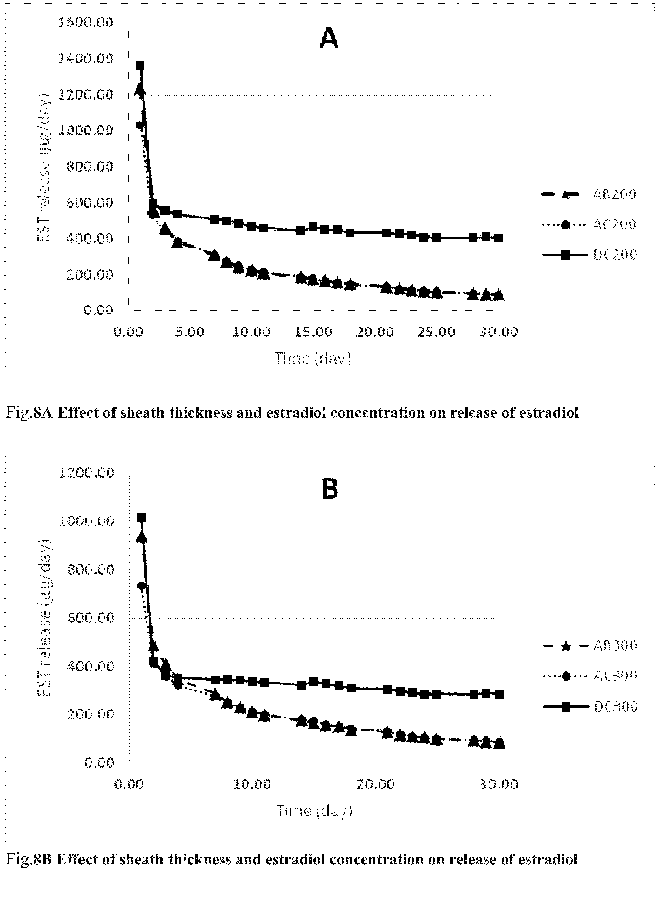

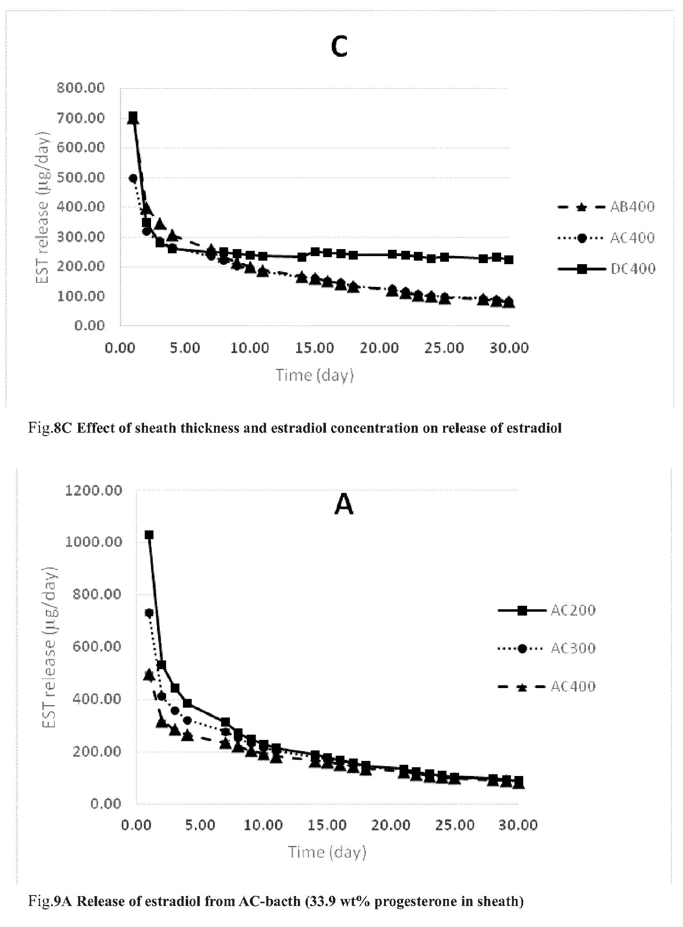

As is evident from Figure 8A, B and C the presence of progesterone in the sheath, reduces the daily release of estradiol.

This effect is less pronounced with low sheath thickness e.g. 200 µm between AB and AC IVRs, where estradiol (0.39 wt%) is mainly dissolved in the core, see Figure 8A.

When the estradiol exists in the core in crystal form (e.g. 10 wt%) estradiol as in the DC batches a maximum release threshold is defined by the sheath thickness loaded with progesterone crystals, as shown in Figure 8A, B and C.

In addition to the dissolved progesterone that hinders the release of estradiol, see Figure 5, the presence of the progesterone in crystal form also has an impact on the release of estradiol as shown in Figure 8.

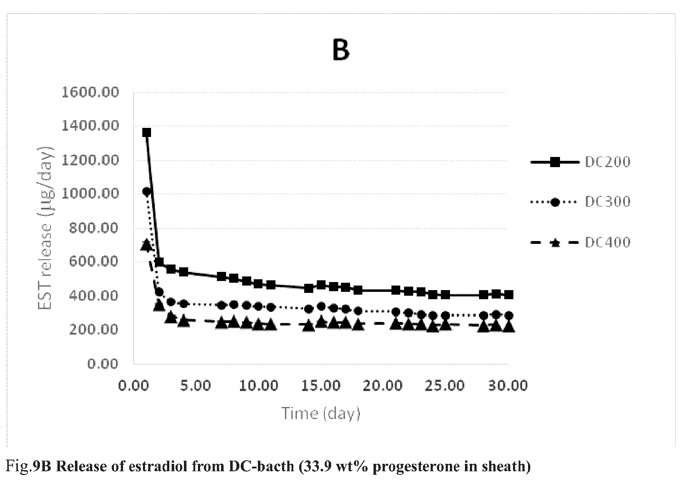

As is evident from Figure 9A and B, the presence of the estradiol in crystal form i.e., the DC-batches with 10 wt% estradiol, allows sustained release of estradiol at zero order release rate of estradiol from the IVRs for 28 days.

There is a slight increase in the release of estradiol after day 14 which may be the result of two physical phenomena; the presence of progesterone crystal in the sheath which tends to increase the diffusion length (i.e., reducing the average release rate) and the depletion of crystals in the sheath which allows faster diffusion through the holes in the sheaths. It is in this respect believed that the space initially occupied by progesterone leaves behind an empty porous matrix (holes) which may be empty or become water-filled due to ingress of water. This means that the rings are in percolation or the crystals are dense near the outer surface of the IVRs.

Thus, it is believed that the space initially occupied by the progestational steroid leaves behind an empty porous matrix which may become water filled due to ingress of water and/or may leave behind empty holes thereby providing a free path for the estradiol, and accordingly the desired zero-order release profile.

The increase in the thickness of the sheath reduces the release rate of the estradiol embedded in the core. The increase of sheath thickness from 200 to 400 µm leads to a decrease in the average daily release of estradiol on day 24 from 409.14 µg to 226.64 µg (for DC200 and DC400 respectively).

The experiments were perform using EVA28 (EVA 28 wt%), where it acted like a rate-controlling sheath. Similar results are expected with other preferred EVAs according to the present invention, e.g. ethylene-vinyl acetate polymer with a vinyl acetate content of 24 wt%, 33 wt% or 40 wt%.

The presence of progesterone limited/tailored the release rate of estradiol and provided a zero-order release of estradiol during the treatment period of 28 when the core is loaded at 10 wt% of estradiol.

Thus, based on the experimental data it can be concluded that the sheath-loaded progesterone crystals play the role of rate-limiting sheath with the tested VA-content. Without being bound by theory, it is in this respect believed that progesterone will function as a filler and control the release rate of estradiol in the core. When progesterone present in the sheath is steroid is reduced and it is believed that that diffusion of water into the sheath may be facilitated leaving behind an empty porous matrix and/or empty pockets/holes and/or the sheath may collapse thereby ensuring the desired zero-order release profile of the estrogenic steroid. Such a zero order release was obtained with the DC-batch (10 wt% estradiol in the core and 33.9 wt% of progesterone in the sheath), see Figure 9B.

Using the drug delivery system, e.g. an intravaginal ring, according to the present invention the inventors have found that it is possible to attain independent and optimal release of the two active ingredients; an estrogenic steroid and an progestational steroid without the need for complex assembly of parts and without the need to use sophisticated multi-layer extrusion technology.

The drug delivery system according to the invention has a simple and inexpensive design, and can therefore be used equally well both privately and in medical or hospital facilities.

## Claims

1. A drug delivery system (1) comprising
- a core (2) comprising a first polymeric material (3) and a first active ingredient (4), and
- a sheath (5) comprising a second polymeric material (6) and a second active ingredient (7) dispersed and/or incorporated in the second polymeric material in a concentration of at least 10 wt% based on the weight of the sheath (5), wherein the first active ingredient (4) and second active ingredient (7) is a steroid, and wherein the steroid is a contraceptive agent, and
- wherein the sheath is the outer layer of the drug delivery system (1).

2. A drug delivery system (1) according to claim 1, wherein the second active ingredient (7) is dispersed and/or incorporated in the second polymeric material (6) in a concentration of at least 15 wt% preferably at least 20 wt%, and even more preferred at least 25 wt%.

3. A drug delivery system (1) according to claim 1 or 2, wherein the second active ingredient (7) is dispersed and/or incorporated in the second polymeric material (6) in the form of particles, such as crystals.

4. A drug delivery system (1) according to any of the preceding claims, wherein the second active ingredient (7) is dispersed and/or incorporated in the second polymeric material (6) in a concentration below the percolation threshold of said second active ingredient in the sheath (5).

5. A drug delivery system (1) according to any of the preceding claims, wherein the second active ingredient (7) is dispersed and/or incorporated in the second polymeric material (6) in a concentration of 40 wt% or below based on the weight of the sheath (5), preferably in a concentration of 35 wt% or below.

6. A drug delivery system (1) according to any of the preceding claims, wherein the first active ingredient (4) is dispersed and/or incorporated and/or dissolved in the first polymeric material (3) in a concentration above 5 wt% based on the weight of the core (2), preferably at least 10 wt% based on the weight of the core, preferably at least 15 wt% based on the weight of the core and even more preferred at least 20 wt% based on the weight of the core.

7. A drug delivery system (1) according to any of the preceding claims, wherein the at least one first active ingredient (4) is dispersed and/or incorporated in the first polymeric material (3) in the form of particles, such as crystals.

8. A drug delivery system (1) according to any of the claims 3 to 7, wherein the particles of the second active ingredient (7) and optionally the first active ingredient (4), have a particle size of 3 µm and 40 µm, preferably between 8 µm and 24 µm, and even more preferred between 10 and 24 µm, as determined by laser diffraction.

9. A drug delivery system (1) according to any of the claims 1 to 6, wherein the first active ingredient (4) is dissolved in the first polymeric material (3) in a concentration below the saturation concentration of said first active ingredient at 25°C.

10. A drug delivery system (1) according to any of the preceding claims, wherein the first polymeric material (3) and/or second polymeric material (6) is at least one inert thermoset or thermoplastic elastomer, such as ethylene-vinyl acetate (EVA) copolymers, low-density polyethylene, polyurethanes, and styrene-butadiene copolymers.

11. A drug delivery system (1) according to any of the preceding claims, wherein the first polymeric material (3) is an ethylene-vinyl acetate copolymer with a vinyl acetate content from 26 to 40 wt%, preferably around 28 wt%, 33 wt% or 40 wt%.

12. A drug delivery system (1) according to any of the preceding claims, wherein the second polymeric material (6) is a ethylene-vinyl acetate copolymer with a vinyl acetate content from 12 to 28 wt%, preferably a vinyl acetate content between 14 and 24 wt%, such as around 20 wt%.

13. A drug delivery system (1) according to any of the preceding claims, wherein the contraceptive agent is an estrogenic steroid, and/or a progestational steroid.

14. A drug delivery system (1) according to to any of the preceding claims, wherein the first active ingredient (4) is a first contraceptive agent, such as estradiol, and the second active ingredient (7) is a different contraceptive agent, such as progesterone.

15. A drug delivery system (1) according to any of the preceding claims, wherein the thickness of the sheath (5) is between 0.05 mm and 3 mm, preferably between 0.05 mm and 2 mm, more preferably between 0.1 mm and 2 mm and even more preferably between 0.1 mm and 0.6 mm.

16. A drug delivery system (1) according to any of the preceding claims, wherein the cross-sectional diameter of the core (2) is from between 2 and 8 mm, more preferably between 3 mm and 6 mm and even more preferably around 4 mm.

17. A method of manufacturing the drug delivery system according to any of the claims 1 - 16, said method comprises
a. providing a core (2) comprising a first polymeric material (3) and a first active ingredient (4),
b. providing a sheath (5) comprising a second polymeric material (6) and a second active ingredient (7) dispersed and/or incorporated in the second polymeric material in a concentration of at least 10 wt% based on the weight of the sheath (5), and
c. co-extruding the core (2) and sheath (5) into a fiber.

18. A method according to claim 17, wherein said method further comprises a cooling step in which the provided drug delivery system is cooled to a temperature of 20°C or below in order to provide crystals of the one second active ingredient (7) in the second polymeric material (6).

19. A method according to claim 18, wherein said cooling step is performed immediately after step c.

20. A method according to any of the claims 17 to 19, wherein the fiber obtained in step c is cut and/or shaped into a drug delivery device.

21. A dual drug delivery device comprising the drug delivery system according to any of the claims 1 - 16 or obtained with the method according to any of the claims 17 - 20, wherein the drug delivery device is in the form of an implant, an intrauterine device or a vaginal ring.

## Patentansprüche

1. Arzneimittelabgabesystem (1), umfassend
- einen Kern (2), der ein erstes Polymermaterial (3) und einen ersten Wirkstoff (4) umfasst, und
- eine Hülle (5), die ein zweites Polymermaterial (6) und einen zweiten Wirkstoff (7) umfasst, der in dem zweiten Polymermaterial in einer Konzentration von mindestens 10 Gew.-%, bezogen auf das Gewicht der Hülle (5), dispergiert und/oder inkorporiert ist, wobei der erste Wirkstoff (4) und der zweite Wirkstoff (7) ein Steroid sind und wobei das Steroid ein empfängnisverhütendes Mittel ist, und
- wobei die Hülle die äußere Schicht des Arzneimittelabgabesystems (1) ist.

2. Arzneimittelabgabesystem (1) nach Anspruch 1, wobei der zweite Wirkstoff (7) in dem zweiten Polymermaterial (6) in einer Konzentration von mindestens 15 Gew.-%, vorzugsweise mindestens 20 Gew.-% und noch bevorzugter mindestens 25 Gew.-% dispergiert und/oder inkorporiert ist.

3. Arzneimittelabgabesystem (1) nach Anspruch 1 oder 2, wobei der zweite Wirkstoff (7) in dem zweiten Polymermaterial (6) in Form von Partikeln, wie etwa Kristallen, dispergiert und/oder inkorporiert ist.

4. Arzneimittelabgabesystem (1) nach einem der vorhergehenden Ansprüche, wobei der zweite Wirkstoff (7) in dem zweiten Polymermaterial (6) in einer Konzentration unterhalb der Perkolationsschwelle des zweiten Wirkstoffs in der Hülle (5) dispergiert und/oder inkorporiert ist.

5. Arzneimittelabgabesystem (1) nach einem der vorhergehenden Ansprüche, wobei der zweite Wirkstoff (7) in dem zweiten Polymermaterial (6) in einer Konzentration von 40 Gew.-% oder weniger, bezogen auf das Gewicht der Hülle (5), vorzugsweise in einer Konzentration von 35 Gew.-% oder weniger, dispergiert und/oder inkorporiert ist.

6. Arzneimittelabgabesystem (1) nach einem der vorhergehenden Ansprüche, wobei der erste Wirkstoff (4) in dem ersten Polymermaterial (3) in einer Konzentration über 5 Gew.-%, bezogen auf das Gewicht des Kerns (2), vorzugsweise mindestens 10 Gew.-%, bezogen auf das Gewicht des Kerns, vorzugsweise mindestens 15 Gew.-%, bezogen auf das Gewicht des Kerns und noch bevorzugter mindestens 20 Gew.-%, bezogen auf das Gewicht des Kerns, dispergiert und/oder inkorporiert und/oder gelöst ist.

7. Arzneimittelabgabesystem (1) nach einem der vorhergehenden Ansprüche, wobei der mindestens eine erste Wirkstoff (4) in dem ersten Polymermaterial (3) in Form von Partikeln, wie etwa Kristallen, dispergiert und/oder inkorporiert ist.

8. Arzneimittelabgabesystem (1) nach einem der Ansprüche 3 bis 7, wobei die Partikel des zweiten Wirkstoffs (7) und gegebenenfalls des ersten Wirkstoffs (4) eine Partikelgröße von 3 µm und 40 µm, vorzugsweise zwischen 8 µm und 24 µm und noch bevorzugter zwischen 10 µm und 24 µm, wie durch Laserbeugung bestimmt, aufweisen.

9. Arzneimittelabgabesystem (1) nach einem der Ansprüche 1 bis 6, wobei der erste Wirkstoff (4) in dem ersten Polymermaterial (3) in einer Konzentration unterhalb der Sättigungskonzentration des ersten Wirkstoffs bei 25 °C gelöst ist.

10. Arzneimittelabgabesystem (1) nach einem der vorhergehenden Ansprüche, wobei das erste Polymermaterial (3) und/oder das zweite Polymermaterial (6) mindestens ein inertes duroplastisches oder thermoplastisches Elastomer, wie etwa Ethylen-Vinylacetat-Copolymere (EVA-Copolymere), Polyethylen niedriger Dichte, Polyurethane und StyrolButadien-Copolymere, ist.

11. Arzneimittelabgabesystem (1) nach einem der vorhergehenden Ansprüche, wobei das erste Polymermaterial (3) ein Ethylen-Vinylacetat-Copolymer mit einem Vinylacetatgehalt von 26 bis 40 Gew.-%, vorzugsweise etwa 28 Gew.-%, 33 Gew.-% oder 40 Gew.-%, ist.

12. Arzneimittelabgabesystem (1) nach einem der vorhergehenden Ansprüche, wobei das zweite Polymermaterial (6) ein Ethylen-Vinylacetat-Copolymer mit einem Vinylacetatgehalt von 12 bis 28 Gew.-%, vorzugsweise einem Vinylacetatgehalt zwischen 14 und 24 Gew.-%, wie etwa 20 Gew.-%, ist.

13. Arzneimittelabgabesystem (1) nach einem der vorhergehenden Ansprüche, wobei das empfängnisverhütende Mittel ein Östrogensteroid und/oder ein Gestagensteroid ist.

14. Arzneimittelabgabesystem (1) nach einem der vorhergehenden Ansprüche, wobei der erste Wirkstoff (4) ein erstes empfängnisverhütendes Mittel, wie etwa Estradiol, ist und der zweite Wirkstoff (7) ein anderes empfängnisverhütendes Mittel, wie etwa Progesteron, ist.

15. Arzneimittelabgabesystem (1) nach einem der vorhergehenden Ansprüche, wobei die Dicke der Hülle (5) zwischen 0,05 mm und 3 mm, vorzugsweise zwischen 0,05 mm und 2 mm, mehr bevorzugt zwischen 0,1 mm und 2 mm und noch mehr bevorzugt zwischen 0,1 mm und 0,6 mm beträgt.

16. Arzneimittelabgabesystem (1) nach einem der vorhergehenden Ansprüche, wobei der Querschnittsdurchmesser des Kerns (2) zwischen 2 und 8 mm, mehr bevorzugt zwischen 3 mm und 6 mm und noch mehr bevorzugt etwa 4 mm beträgt.

17. Verfahren zur Herstellung des Arzneimittelabgabesystems nach einem der Ansprüche 1 bis 16, wobei das Verfahren Folgendes umfasst:
a. Bereitstellen eines Kerns (2), der ein erstes Polymermaterial (3) und einen ersten Wirkstoff (4) umfasst,
b. Bereitstellen einer Hülle (5), die ein zweites Polymermaterial (6) und einen zweiten Wirkstoff (7) umfasst, der in dem zweiten Polymermaterial in einer Konzentration von mindestens 10 Gew.-%, bezogen auf das Gewicht der Hülle (5), dispergiert und/oder inkorporiert ist, und
c. Coextrudieren des Kerns (2) und der Hülle (5) zu einer Faser.

18. Verfahren nach Anspruch 17, wobei das Verfahren ferner einen Kühlschritt umfasst, in dem das bereitgestellte Arzneimittelabgabesystem auf eine Temperatur von 20 °C oder darunter gekühlt wird, um Kristalle des einen zweiten Wirkstoffs (7) in dem zweiten Polymermaterial (6) bereitzustellen.

19. Verfahren nach Anspruch 18, wobei der Kühlschritt unmittelbar nach Schritt c durchgeführt wird.

20. Verfahren nach einem der Ansprüche 17 bis 19, wobei die in Schritt c erhaltene Faser zu einer Arzneimittelabgabevorrichtung geschnitten und/oder geformt wird.

21. Duale Arzneimittelabgabevorrichtung, umfassend das Arzneimittelabgabesystem nach einem der Ansprüche 1 bis 16 oder erhalten mit dem Verfahren nach einem der Ansprüche 17 bis 20, wobei die Arzneimittelabgabevorrichtung in Form eines Implantats, einer Intrauterinpession oder eines Vaginalrings vorliegt.

## Revendications

1. Un système d'administration de médicaments (1) comprenant
- un noyau (2) comprenant un premier matériau polymère (3) et un premier ingrédient actif (4), et
- une gaine (5) comprenant un deuxième matériau polymère (6) et un deuxième ingrédient actif (7) dispersé et/ou incorporé dans le deuxième matériau polymère à une concentration d'au moins 10 % en poids par rapport au poids de la gaine (5), où le premier ingrédient actif (4) et le deuxième ingrédient actif (7) sont des stéroïdes, et où le stéroïde est un agent contraceptif, et
- où la gaine est la couche extérieure du système d'administration de médicaments (1).

2. Un système d'administration de médicaments (1) selon la revendication 1, où le deuxième ingrédient actif (7) est dispersé et/ou incorporé dans le deuxième matériau polymère (6) à une concentration d'au moins 15 % en poids, de préférence au moins 20 %, et encore plus préférablement au moins 25 %.

3. Un système d'administration de médicaments (1) selon la revendication 1 ou 2, où le deuxième ingrédient actif (7) est dispersé et/ou incorporé dans le deuxième matériau polymère (6) sous forme de particules, telles que des cristaux.

4. Un système d'administration de médicaments (1) selon l'une quelconque des revendications précédentes, où le deuxième ingrédient actif (7) est dispersé et/ou incorporé dans le deuxième matériau polymère (6) à une concentration inférieure au seuil de percolation dudit deuxième ingrédient actif dans la gaine (5).

5. Un système d'administration de médicaments (1) selon l'une quelconque des revendications précédentes, où le deuxième ingrédient actif (7) est dispersé et/ou incorporé dans le deuxième matériau polymère (6) à une concentration de 40 % en poids ou moins par rapport au poids de la gaine (5), de préférence à une concentration de 35 % en poids ou moins.

6. Un système d'administration de médicaments (1) selon l'une quelconque des revendications précédentes, où le premier ingrédient actif (4) est dispersé et/ou incorporé et/ou dissous dans le premier matériau polymère (3) à une concentration supérieure à 5 % en poids par rapport au poids du noyau (2), de préférence au moins 10 % en poids par rapport au poids du noyau, de préférence au moins 15 % en poids par rapport au poids du noyau et encore plus préférablement au moins 20 % en poids par rapport au poids du noyau.

7. Un système d'administration de médicaments (1) selon l'une quelconque des revendications précédentes, où le premier ingrédient actif (4) est dispersé et/ou incorporé dans le premier matériau polymère (3) sous forme de particules, telles que des cristaux.

8. Un système d'administration de médicaments (1) selon l'une quelconque des revendications 3 à 7, où les particules du deuxième ingrédient actif (7) et éventuellement du premier ingrédient actif (4), ont une taille de particule de 3 µm à 40 µm, de préférence entre 8 µm et 24 µm, et encore plus préférablement entre 10 µm et 24 µm, déterminée par diffraction laser.

9. Un système d'administration de médicaments (1) selon l'une quelconque des revendications 1 à 6, où le premier ingrédient actif (4) est dissous dans le premier matériau polymère (3) à une concentration inférieure à la concentration de saturation dudit premier ingrédient actif à 25°C.

10. Un système d'administration de médicaments (1) selon l'une quelconque des revendications précédentes, où le premier matériau polymère (3) et/ou le deuxième matériau polymère (6) est au moins un élastomère thermodurcissable ou thermoplastique inerte, tel que des copolymères d'éthylène-acétate de vinyle (EVA), du polyéthylène basse densité, des polyuréthanes et des copolymères de styrène-butadiène.

11. Un système d'administration de médicaments (1) selon l'une quelconque des revendications précédentes, où le premier matériau polymère (3) est un copolymère d'éthylène-acétate de vinyle avec une teneur en acétate de vinyle de 26 à 40 % en poids, de préférence autour de 28 % en poids, 33 % en poids ou 40 % en poids.

12. Un système d'administration de médicaments (1) selon l'une quelconque des revendications précédentes, où le deuxième matériau polymère (6) est un copolymère d'éthylène-acétate de vinyle avec une teneur en acétate de vinyle de 12 à 28 % en poids, de préférence une teneur en acétate de vinyle entre 14 et 24 % en poids, telle qu'environ 20 % en poids.

13. Un système d'administration de médicaments (1) selon l'une quelconque des revendications précédentes, où l'agent contraceptif est un stéroïde œstrogénique, et/ou un stéroïde progestatif.

14. Un système d'administration de médicaments (1) selon l'une quelconque des revendications précédentes, où le premier ingrédient actif (4) est un premier agent contraceptif, tel que l'estradiol, et le deuxième ingrédient actif (7) est un agent contraceptif différent, tel que la progestérone.

15. Un système d'administration de médicaments (1) selon l'une quelconque des revendications précédentes, où l'épaisseur de la gaine (5) est comprise entre 0,05 mm et 3 mm, de préférence entre 0,05 mm et 2 mm, plus préférablement entre 0,1 mm et 2 mm et encore plus préférablement entre 0,1 mm et 0,6 mm.

16. Un système d'administration de médicaments (1) selon l'une quelconque des revendications précédentes, où le diamètre transversal du noyau (2) est compris entre 2 mm et 8 mm, plus préférablement entre 3 mm et 6 mm et encore plus préférablement autour de 4 mm.

17. Un procédé de fabrication du système d'administration de médicaments selon l'une quelconque des revendications 1 à 16, ledit procédé comprenant
a. la fourniture d'un noyau (2) comprenant un premier matériau polymère (3) et un premier ingrédient actif (4),
b. la fourniture d'une gaine (5) comprenant un deuxième matériau polymère (6) et un deuxième ingrédient actif (7) dispersé et/ou incorporé dans le deuxième matériau polymère à une concentration d'au moins 10 % en poids par rapport au poids de la gaine (5), et
c. la co-extrusion du noyau (2) et de la gaine (5) en une fibre.

18. Un procédé selon la revendication 17, où ledit procédé comprend en outre une étape de refroidissement dans laquelle le système d'administration de médicaments fourni est refroidi à une température de 20°C ou moins afin de fournir des cristaux du deuxième ingrédient actif (7) dans le deuxième matériau polymère (6).

19. Un procédé selon la revendication 18, où ladite étape de refroidissement est effectuée immédiatement après l'étape c.

20. Un procédé selon l'une quelconque des revendications 17 à 19, où la fibre obtenue à l'étape c est coupée et/ou façonnée en un dispositif d'administration de médicaments.

21. Un dispositif d'administration de médicaments double comprenant le système d'administration de médicaments selon l'une quelconque des revendications 1 à 16 ou obtenu par le procédé selon l'une quelconque des revendications 17 à 20, où le dispositif d'administration de médicaments est sous forme d'implant, de dispositif intra-utérin ou d'anneau vaginal.
